# EUROPEAN PATENT SPECIFICATION

(11) **EP 4 065 046 B1**
(45) Date of publication and mention of the grant of the patent: **23.07.2025**
(21) Application number: 20824825.2
(22) Date of filing: 19.11.2020
(51) Int. Cl.: A61F 2/24, A61B 17/04, A61B 17/00, A61B 1/005, A61B 90/00, A61B 34/20

(54) **PAPILLARY APPROXIMATION TOOL WITH ENHANCED VISUALIZATION**
PAPILLAR-NÄHERUNGS-WERKZEUG MIT VERBESSERTER VISUALISIERUNG
OUTIL D'APPROXIMATION PAPILLAIRE À VISUALISATION AMÉLIORÉE

(30) Priority: 27.11.2019 US 201962941006 P
(43) Date of publication of application: 05.10.2022
(73) Proprietor: Boston Scientific Scimed, Inc., Maple Grove, MN 55311 (US)
(72) Inventor: HOU, Dongming, Plymouth, Minnesota 55446 (US); CICERONE, Raffaele, Galway, H65 A622 (IE); CLARK, Bryan A., Forest Lake, Minnesota 55025 (US); FLANAGAN, Aiden, Galway (IE); O'CONNOR, Tim, Kiniska Claregalway County Galway (IE)
(74) Representative: Peterreins Schley
(86) International application number: PCT/US2020/061275
(87) International publication number: WO 2021/108213

(56) References cited:
- US-A1- 2007 015 964
- US-A1- 2011 060 407
- US-A1- 2013 023 731
- US-A1- 2014 296 875
- US-A1- 2019 298 332

## Description

### FIELD

The present disclosure relates generally to the field of implantable medical devices and more particularly to implantable devices, systems, and methods for adjusting heart features.

### BACKGROUND

Mitral regurgitation (MR) (also referred to as mitral insufficiency, or mitral incompetence) is a form of valvular heart disease in which the leaflets of the mitral valve fail to properly coapt, or close. When the mitral valve does not close properly, blood may be regurgitated; e.g. flow backwards from the left ventricle to the left atrium, leading to cardiac deformation wherein the mitral annulus and/or chambers of the heart may thicken and/or become enlarged, further exacerbating regurgitation. Atrial fibrillation, congestive heart failure, cardiogenic shock, and other adverse events may occur as a result.

Mitral valve repair may include a combination of annular and sub-valvular procedures intended to restore the physiological form and function of the mitral valve. For example, annuloplasty procedures may involve surgically implanting a ring around the mitral annulus to restore a diameter of the patient's mitral annulus to that of a healthy state where the valve leaflets properly coapt and mitral regurgitate flow is minimized. Additionally, sub-valvular repair procedures such as repositioning of papillary muscles or repairing chordae within the left ventricle may be performed.

Due to the invasive nature of the surgical approaches to mitral valve repair, several transcatheter techniques have been developed to emulate surgical approaches. Because delivery catheters that carry mitral valve or sub-valvular components may extend up to 52" in length, it can be challenging to transport and accurately place repair components at a treatment site.

US 2013/023731 A1 discloses a tissue visualization device for facilitating clearing of opaque bodily fluids. US 2007/015964 A1 discloses an apparatus for imaging a body structure within a body cavity. US 2014/0296875 A1 discloses a flexible sheath instrument and a flexible guide instrument.

### SUMMARY

The present invention is directed to a delivery system as set forth in the appended claims. According to the invention, the delivery system includes a visualization catheter including a proximal end, a distal end, and an elongate tubular body extending from the proximal end to the distal end of the visualization catheter, the visualization catheter including an imaging device coupled to a distal portion of the visualization catheter. An anchor lumen extends from the proximal end of the visualization catheter through an anchor port disposed on a distal wall of the visualization catheter proximate to its distal end. The delivery system includes an adjustment mechanism, translatably disposed within a lumen of the visualization catheter, the adjustment mechanism configured to align the anchor port with a tissue target based on feedback provided by the imaging device.

In various embodiments, the imaging device includes an ultrasound transducer. In one embodiment, the adjustment mechanism includes a distal anchor. The delivery system may further include a retention mechanism, disposed about the visualization catheter, the retention mechanism including a linear configuration where the retention mechanism may be flush with the visualization catheter and an expanded configuration where the retention mechanism extends radially from the visualization catheter towards cardiac tissue. In some embodiments, the retention mechanism may include a balloon, and the balloon may be coupled to an inflation lumen of the visualization catheter. In some embodiments, the retention mechanism may include a plurality of expandable splines. In some embodiments, the retention mechanism may include an expandable stent. In various embodiments, the retention mechanism may be disposed proximate to or about at least a portion of the imaging device. In some embodiments, the delivery system may further include a needle, translatably disposed within the anchor lumen, the needle including a sharpened distal tip and having a needle lumen extending therethrough, an anchor delivery catheter, translatably disposed within the needle lumen, an anchor, disposed within the anchor delivery catheter and a push rod, translatably disposed within the anchor delivery catheter proximally of the anchor, the push rod configured to advance the anchor through the distal end of the anchor delivery catheter into the tissue target. In some embodiments, the anchor may be one of a plurality of anchors, disposed within the anchor lumen, where each anchor may be coupled to a distal end of one of a plurality of sutures, the proximal end of the sutures extending proximally through the anchor lumen, where the visualization catheter is rotatable about the adjustment mechanism to direct the anchor port towards a plurality of different tissue targets to embed the plurality of anchors within the different tissue targets, and where the delivery system includes a clamping mechanism, translatably disposed within a working channel of the visualization catheter, the clamping mechanism configured to join at least two of the plurality of sutures.

The present disclosure also provides a system for delivering repair components to a cardiac cavity includes a visualization catheter including a proximal end, a distal end, and an elongate tubular body extending from the proximal end to the distal end of the visualization catheter, the visualization catheter including an imaging device coupled to a distal portion of the visualization catheter. The system includes at least one stabilization mechanism for positioning a distal end of the visualization catheter within the cardiac cavity and an anchor delivery system, disposed within an anchor lumen of the visualization catheter. In some embodiments, the anchor lumen extends from the proximal end of the visualization catheter through an anchor port disposed on a distal wall of the visualization catheter and the anchor delivery system includes a hollow needle, translatably disposed within the anchor lumen. The anchor delivery system includes an anchor delivery catheter, translatably disposed within the hollow needle, and an anchor having a sharpened distal end and a proximal coupler coupled to a suture, the anchor translatably disposed within the anchor delivery catheter. The system also includes an actuator configured to expel the anchor from the anchor delivery catheter to embed the anchor into tissue.

In various embodiments, the visualization catheter further includes a working channel and a clamp tool disposed within the working channel. The stabilization mechanism may include a retention mechanism such as a balloon, a spline, a stent or a combination thereof. In some embodiments, the retention mechanism includes a linear configuration where the retention mechanism lies flush against an external surface of the visualization catheter and an expanded configuration where at least a portion of the retention mechanism extends radially from the external surface of the visualization catheter. In some embodiments, the stabilization mechanism includes a depth adjustment mechanism, translatably disposed within a lumen of the visualization catheter and configured to extend distally of the visualization catheter to control a depth of the anchor port.

In some embodiments, the anchor lumen may be one of a plurality of anchor lumens, each anchor lumen of the plurality of anchor lumens extending from one of a plurality of ports disposed on the distal portion of the visualization catheter to the proximal end of the visualization catheter. The plurality of ports may include at least two ports disposed along a common longitudinal path or along different longitudinal paths or both. In some embodiments, the system further includes a radio opaque marker disposed upon the distal portion of the visualization catheter or the hollow needle or the anchor delivery catheter or a combination thereof.

The present disclosure also provides a method of papillary approximation includes advancing a distal portion of a visualization catheter into a heart chamber, the visualization catheter including a stabilization mechanism disposed within a lumen and activating the stabilization mechanism to secure the distal portion of the visualization catheter within the heart chamber. The method includes imaging the heart chamber to identify a first tissue target and adjusting the stabilization mechanism to align a port extending through a wall of the distal portion of the visualization catheter with the first tissue target. The method includes advancing a hollow needle through the port into the first tissue target and forwarding a first anchor through the hollow needle into the first tissue target, the first anchor coupled at a proximal end to a first suture. The method includes rotating the visualization catheter, imaging the heart chamber to identify a second tissue target and advancing the hollow needle through the port into the second tissue target. The method includes forwarding a second anchor through the hollow needle into the second tissue target, the second anchor coupled at a proximal end to a second suture, pulling together the first suture and the second suture to pull the first anchor and first tissue target towards the second anchor and second tissue target to provide a modified heart chamber configuration and clamping the first suture to the second suture to retain the modified heart chamber configuration.

In various embodiments, the port is one of a plurality of ports disposed along and through the distal portion of the visualization catheter, and advancing the hollow needle into the first tissue target and advancing the hollow needle into the second tissue target uses different ones of the plurality of ports. In other embodiments, the first anchor may be one of a first pair of anchors, the second anchor may be one of a second pair of anchors, and the method includes forwarding the first pair of anchors through a pair of ports into the first tissue target, rotating the visualization catheter and forwarding the second pair of anchors through the pair of ports into the second tissue target. With such an arrangement, an implant and method of delivery is disclosed which enables non-invasive accurate placement of sub-valvular repair components.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical illustrated component is typically represented by a single numeral. For purposes of clarity, not every component is labeled in every figure, nor is every component of each embodiment shown where illustration is not necessary to allow those of ordinary skill in the art to understand the disclosure. In the figures:
FIG. 1 is a diagram of a portion of a heart in which delivery systems for sub-valvular repair such as those disclosed in various embodiments herein may be deployed;
FIG. 2 is a diagram of one embodiment of a visualization catheter as disclosed herein;
FIG. 3 is a cross-section diagram of one embodiment of an anchor delivery system as disclosed herein;
FIGS. 4A and 4B are cross-sectional views of a distal portion of one embodiment of a visualization catheter as disclosed herein;
FIGS. 5A-5G are views of left chambers of a heart used to illustrate examples of steps that may be performed during a sub-valvular repair procedure disclosed herein;
FIGS. 6A-6D illustrate examples of steps that may be performed by one embodiment of an anchor delivery system during the papillary approximation procedure described in FIGS. 5A-5G;
FIG. 7 is one embodiment of a spline-based retention mechanism that may be used with the visualization catheter disclosed herein; and
FIG. 8 is one embodiment of a stent-based retention mechanism that may be used with the visualization catheter disclosed herein.

### DETAILED DESCRIPTION

The present invention is directed to a delivery system as set forth in the appended claims. A device, system, and method enabling sub-valvular repair of papillary and/or other myocardial structure to an approximately healthy configuration is described herein. For example, such a device, system, and method may be used when mitral valve regurgitation (MR) enlarges the chambers of the heart, displacing papillary muscles and impairing the function of the mitral valve. According to one embodiment a system for controlled delivery of sub-valvular restructuring components includes a visualization catheter supporting an imaging device, such as an ultrasound transducer, that may be used to visualize sub-valvular placement of anchors to improve restructuring accuracy. The visualization catheter includes one or more anchor lumens, extending from a proximal end of the visualization catheter through a port disposed on a distal wall of the visualization catheter, proximate to the imaging device. The imaging device may be used to identify a tissue target, for example in the papillary muscles, ventricle wall, and/or other myocardial tissue, and to guide the deployment of the anchor into the tissue target with increased accuracy. In some embodiments, the visualization catheter may also include one or more stabilization mechanisms that secure the visualization catheter within the heart chamber during anchor deployment. The stabilization mechanism may include, for example, a depth adjustment mechanism, which can be controlled to align the port of the visualization catheter with one or more tissue targets. The ability to control the depth of anchor placement using such an alignment mechanism may reduce vector offsets between pairs of deployed anchors, thereby increasing anchoring accuracy and producing a more natural structure. In some embodiments, the stabilization mechanism may include a retention mechanism. The retention mechanism may include an expandable member that may be disposed about the distal portion of the visualization catheter and may act to anchor the distal portion of the visualization catheter between tissue structures of the ventricle. The retention mechanism may also serve to reduce entanglement with the chordae tendinea and other structures within the ventricle.

These and other beneficial aspects of a system for sub-valvular repair are described in more detail below. It should be noted that, although embodiments of the present disclosure may be described with specific reference to papillary muscles, the principles disclosed herein may be readily adapted to benefit any other dilatation, valve incompetency, valve leakage, and other similar heart failure conditions.

As used herein, the term "distal" refers to the end farthest away from the medical professional when introducing a medical device into a patient, while the term "proximal" refers to the end closest to the medical professional when introducing a medical device into a patient.

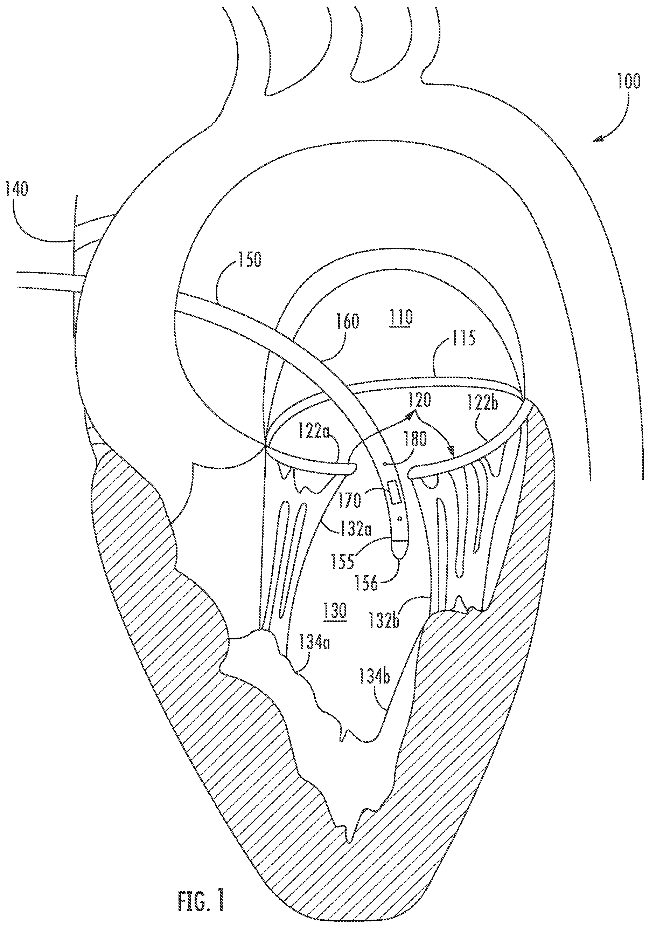

FIG. 1 is a cross-section diagram of a left chamber of a heart 100, including a left atrium 110 separated from a left ventricle 130 by mitral valve 120. The mitral valve 120 includes an anterior leaflet 122a and a posterior leaflet 122b which are attached in a healthy heart to respective papillary muscles 134a, 134b via chordae tendineae 132a, 132b. The papillary muscles 134a, 134b contract to prevent inversion or prolapse of the leaflets 122a, 122b on contraction of the left ventricle 130. A mitral annulus 115 comprises a fibrous ring that, in a healthy heart is saddle shaped and of a diameter to enable the valves to close, or coapt, during systolic contraction.

In a diseased heart, one or more of the chordae tendineae 132a, 132b may be stretched or ruptured, resulting in a flailing leaflet 122a, 122b that no longer effectively closes, resulting in regurgitation. The papillary muscles 134a, 134b may become spaced apart, for example due to enlargement of the heart. Alternatively, or in conjunction, the mitral annulus 115 may become stretched or deformed, and the valves may also fail to close as a result.

To repair the heart failure condition, repair components may be transluminally deployed to the heart 100. In FIG. 1, a delivery system 150 as disclosed herein is shown advanced transseptally introduced through the septum 140, down through the mitral valve 120 and into the left ventricle 130. Depending upon the heart feature that is to be repaired it is appreciated that the present disclosure is not limited by the way the delivery system is introduced to the heart 100. For example, transapical and retrograde aortic delivery methods are within the scope of this disclosure.

In one embodiment, the delivery system 150 may include a plurality of nested catheters including a visualization catheter 160 having a steerable distal end 155 to facilitate navigation of repair components into the left ventricle. In some embodiments, initial delivery of the system 150 may be performed with assistance of a guidewire 156, which may be translatably disposed within a working channel of the visualization catheter 160. According to one aspect, as described in more detail below, the visualization catheter 160 comprises an elongate, generally tubular body having a plurality of lumens extending at least partially from a proximal end to a distal end, and an imaging device 170 disposed proximate to its the distal end. The visualization catheter also includes one or more ports, such as port 180, extending through a wall in the distal portion of the visualization catheter into an anchor lumen that extends through the visualization catheter. In the illustrated embodiment, the ports 180 are shown disposed about the imaging device 170 although the disclosure is not so limited. For example, in other embodiments, the ports may all be disposed proximal or distal to the imaging device 170. As described below, anchors may be driven through the anchor lumen(s) of the visualization catheter, out of the ports and into heart tissue.

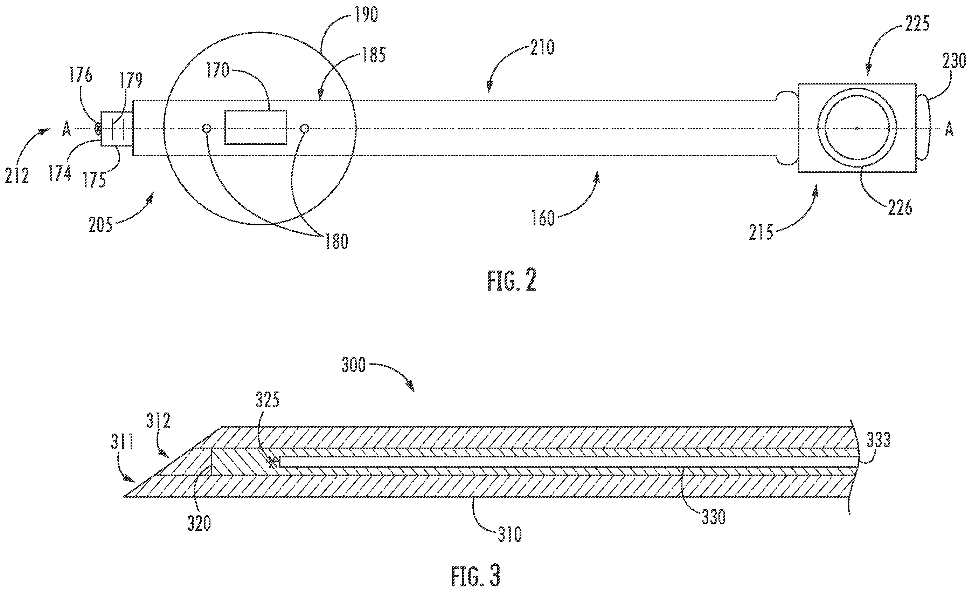

FIG. 2 illustrates the visualization catheter 160 in accordance with the invention in more detail. The visualization catheter 160 includes a tubular body having a proximal end 215, a distal end 205, and an elongate body 210 extending therebetween. A lumen 212 may extend from the proximal end 215 through the distal end 205 of the visualization catheter 160 along axis 'A' in FIG. 2. A connector 225 may be disposed at the proximal end 215. In some embodiments, the connector 225 may include control, such as dial 226 for steering the distal end 205 of the catheter 160. The proximal end 215 may further include a port 230, enabling introduction of one or more tools, needles, or the like into lumens of the catheter 160.

In some embodiments, the elongate body 210 of the visualization catheter 160 may comprise a composite of layers of thermoplastic elastomer (TPE), for example PEBAX provided by ARKEMA corporation of Colombes France. Alternatively, nylon, polyurethanes, polyester, silicone, or other similar materials may be used to provide thin walls that may be extruded and layered over braided wires or coils for tensile and hoop strength, although the disclosed system is not limited to any particular material composition. In some embodiments, the length of the visualization catheter 160 may range from between 24"-52", and more particularly between 42"-46".

The visualization catheter 160 is shown to include an imaging device 170 disposed proximate to its distal end. The imaging device 170 may comprise, for example, an ultrasound transducer. The ultrasound transducer may be directly coupled to the visualization catheter, for example, attached to an external surface or formed at least partially within the visualization catheter. Alternatively, the ultrasound transducer may be indirectly coupled to the visualization catheter, for example coupled to or disposed upon a stabilization mechanism, adjustment mechanism and/or retention mechanism of the visualization catheter as described in more detail below. In some embodiments, the ultrasound transducer may be introduced through a channel of the visualization catheter towards the distal end to enable visualization during sub-valvular repair.

The imaging device may operate using any number of scanning modes, including electronic curved linear array, forward-viewing, electronic curved linear array, electronic 360° radial array, mechanical radial, and/or mechanical helical for dual plane reconstruction (DPR). Other ultrasound imaging methods may be substituted herein by those of skill in the art and are considered to be within the scope of this disclosure.

Although one imaging device 170 is shown, in some embodiments multiple imaging devices, such as ultrasound transducers, may be used to enable 3D imaging of the heart cavity. In some embodiments, multiple different types of imaging sensors may be disposed on, within or proximate to the distal end of the visualization catheter, and the image data may be combined to provide an anatomical map with increased detail and complexity. Such imaging devices include, but are not limited to, biopotential or impedance sensors, electromagnetic sensors, intracardiac echocardiography (ICE) imaging sensors, etc. For example, if a 6 degree-of-freedom electromagnetic sensor is incorporated into the catheter, if also coupled with a pre-operative scan such as CT or MR, the catheter position and orientation may be tracked relative to anatomical features as the catheter is advanced and positioned in the heart. When positioned in the heart near expected anatomical landmarks such as the papillary muscle, the ultrasound sensor may be activated, providing real-time anatomical information. All data sources may be combined or fused to provide increased detail and accuracy for positioning of anchors.

In one embodiment the visualization catheter 160 includes one or more stabilization mechanisms which operate to retain the visualization catheter in a relatively consistent position and orientation within the ventricle during sub-valvular reconstruction. For example, a depth adjustment mechanism 175 is translatably disposed within a lumen of the visualization catheter 160. In one embodiment, the depth adjustment mechanism 175 is configured for distal advancement past the distal end 205 of the visualization catheter 160, to enable the distal end 174 of the depth adjustment mechanism 175 to contact the apex (or other interior surface of the heart), to adjust the height of the ports 180 within the heart chamber. Thus, the depth adjustment mechanism 175 may be used to align the ports 180 with target tissue within the heart for anchoring purposes.

**In** some embodiments, the distal end 174 of the depth adjustment mechanism 175 may include an anchor 176, to further secure the catheter 160 within the heart chamber. Other methods of securing the depth adjustment, such as suction or the like, are considered within the scope of this disclosure. **In** some embodiments, the distal end 174 may be formed of or include a flexible material and/or a cushion, configured to control the interaction of the anchor with the heart features to reduce risks of perforation or other damage to cardiac tissue.

In some embodiments, once ports are aligned with target tissue, the position of the depth adjustment mechanism may be locked (for example, using a clamp, threaded collar, etc.) by a lock mechanism (not shown) at the proximal end of the visualization catheter. One advantage provided by the depth adjustment mechanism is that it enables radial deployment of anchors through a common port to align along an axis perpendicular to the visualization catheter. For example, a first anchor may be deployed through a port, the visualization catheter may be rotated, and a second anchor may be deployed through the same port. By maintaining the same depth adjustment for each deployed anchor, the anchors may be aligned along a common circumference, minimizing vector offsets that could reduce the efficacy of the sub-valvular repair procedure.

In some embodiments, a distal end of the depth adjustment mechanism 175 may include one or more markers, such as markers 179 which may be formed, for example, from metal, calcium or other material that is visible via ultrasound for example echogenic coatings or textured surfaces that reflect under echo, such as the Sono-coat^{™} coating provided by Encapson BV of Institutenweg, Enschede, The Netherlands, or other suppliers of echogenic coatings. Such markers may be used, for example, to provide information to the surgeon regarding the height of the ports. Alternatively, or in addition, markings may be provided proximate to, around or partially around ports 180 to further assist with alignment of the ports with tissue targets.

In one embodiment, the stabilization mechanisms of the visualization catheter 160 may also include a retention mechanism 190, such as the balloon illustrated in FIG. 2. **In** one embodiment, the retention mechanism 190 comprises a flexible member having a first, generally linear configuration wherein the retention mechanism lies flush against the external surface of the visualization catheter 160. The retention mechanism 190 may be in the linear configuration for transseptal delivery of the visualization catheter 160 into the ventricle. The retention mechanism may include an expanded configuration (shown in FIG. 2) wherein at least a portion of the retention mechanism 190 extends radially outward from the external surface of the visualization catheter 160. In one embodiment, the retention mechanism helps to stabilize the distal portion of the visualization catheter 160 during anchor delivery. For balloon type retention mechanisms, the balloon may be inflated using a gas or liquid (such as saline), delivered into the balloon from an inflation port 185 coupled to an inflation lumen of the visualization catheter 160. In some embodiments, the balloons may be segmented (for example, along a longitudinal axis of the visualization catheter), or have openings extending therethrough, to enable passage of the anchors from the ports through the balloon and into tissue.

FIG. 2 also illustrates a plurality of ports 180, through which anchors may be delivered for sub-valvular repair. Although two ports are shown in FIG. 2, it is appreciated that there may be only one port 180 or there may be many ports. Each port 180 provides an outlet for an anchor lumen of the visualization catheter 160, and thus it is appreciated that the number of ports is a matter of design choice, limited only by the size of the catheter. Although two ports 180 are shown along a common linear axis of the elongate body 210, it is appreciated that different ports may be disposed in different locations around the elongate body 210, for example, on opposing sides of the elongate body 210. Accordingly, the disclosure is not limited to any particular number or placement of ports for deploying anchors.

An anchor delivery system may be used to forward an anchor through an anchor lumen of the visualization catheter 160 and out of a port 180 into target tissue, under visualization using the ultrasound imaging device 170. One such anchor delivery system 300 is shown in cross section in FIG. 3 to include a needle 310 having a sharpened distal tip 311 and a needle lumen 312 extending therethrough. An anchor catheter 320 is translatably disposed within the needle lumen 312. The anchor catheter 320 is shown to carry, at its distal end, an anchor 325. As described in more detail below, the anchor catheter 320 may be distally translated into target tissue, and the anchor 325 may then be pushed through the anchor catheter 320 into the tissue, for example, by action of a push tube 330. In one embodiment, the anchor 325 may be coupled to a suture 333, that extends proximally through the anchor catheter (external to or within the push tube as shown).

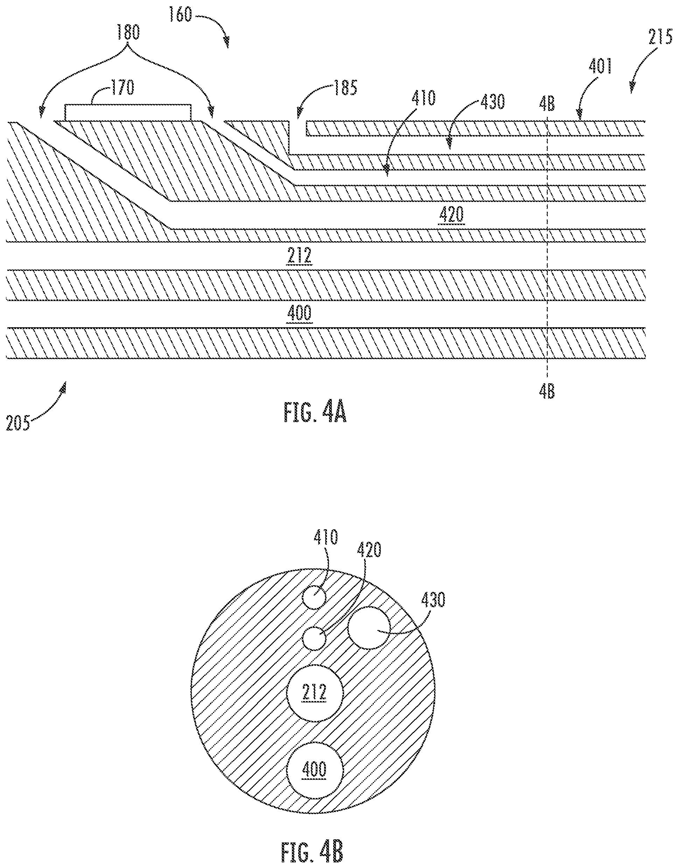

FIG. 4A is a cross section view of the distal end 205 of the visualization catheter 160, including an imaging device 170 and illustrating various lumens that extend through the catheter. In some embodiments, the lumens may comprise defined channels within a unitary body as shown in FIG. 4A, and in alternate embodiments, the lumens may be formed of a plurality of elongate tubes disposed within a sheath. Anchor lumens 410 and 420 are shown to extend from the proximal end 215, deflecting towards an external wall 401 of the visualization catheter 160 as the lumens extend distally towards ports 180. Inflation lumen 430 may provide a conduit for a gas or liquid through port 185, for example that may be used to inflate / deflate the retention mechanism (balloon) 190 (FIG. 2). The visualization catheter 160 also includes a central lumen 212, for example, providing translatable support of the depth adjustment mechanism, and one or more working channels, such as channel 400, which may be used to advance tools (e.g., clamps, ablation devices, suction, etc.) into the treatment site. FIG. 4B is a cross section of the visualization catheter 160 taken along line 4B-4B of FIG. 4A, and illustrating the channels for the anchor lumens 410, 420 and inflation lumen 430 as well as central lumen 212 and working channel 400.

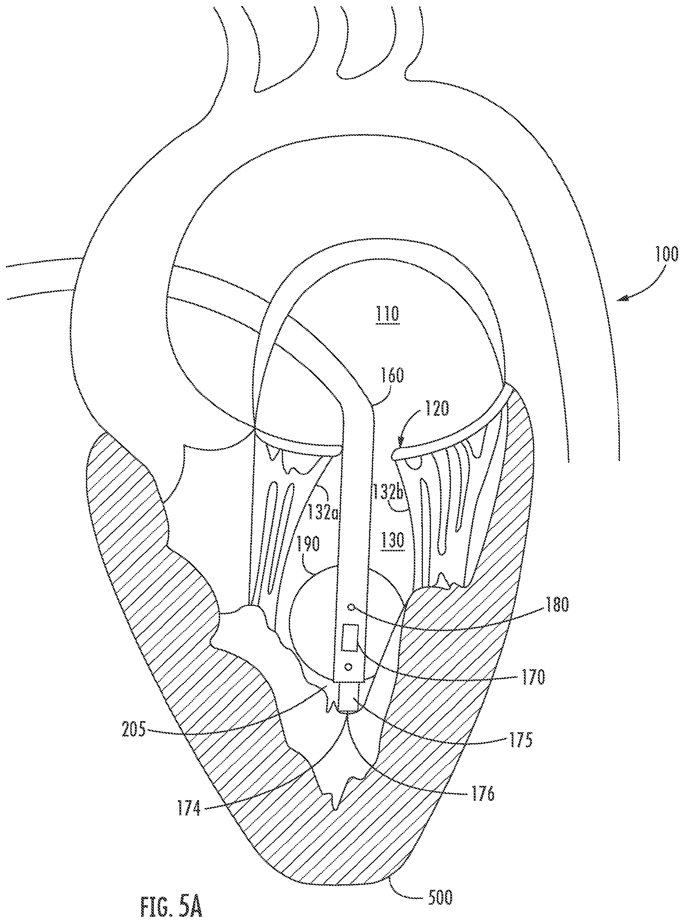

A method for using the embodiment of the delivery system as described above for sub-valvular repair will now be described with regard to FIGS. 5A-5G. In FIG. 5A, the visualization catheter 160 is advanced transseptally into the left atrium 110 and through the mitral valve 120 into the left ventricle 130. The imaging device 170 is used to visualize the myocardial tissue, to target tissue, and the depth adjustment mechanism 175 is advanced distally from the distal end 205 of the visualization catheter until it contacts the inner wall of the apex 500 of the heart 100. Anchor 176 may be driven into the tissue of the apex 500, and the relative position between the distal end 174 of the depth adjustment mechanism 175 and the distal end 205 of the visualization catheter 160 adjusted until the port 180 aligns with the target tissue. When alignment is achieved, the depth adjustment mechanism 175 may be locked in place, inhibiting further distal translation of the visualization catheter 160 within the heart 100. Fluid may then be dispensed into the retention mechanism 190 until the retention mechanism contacts myocardial tissue, further securing the visualization catheter in place and protecting against interference from the chordae tendinea 132a, 132b and the anchor deployment system of the visualization catheter 160.

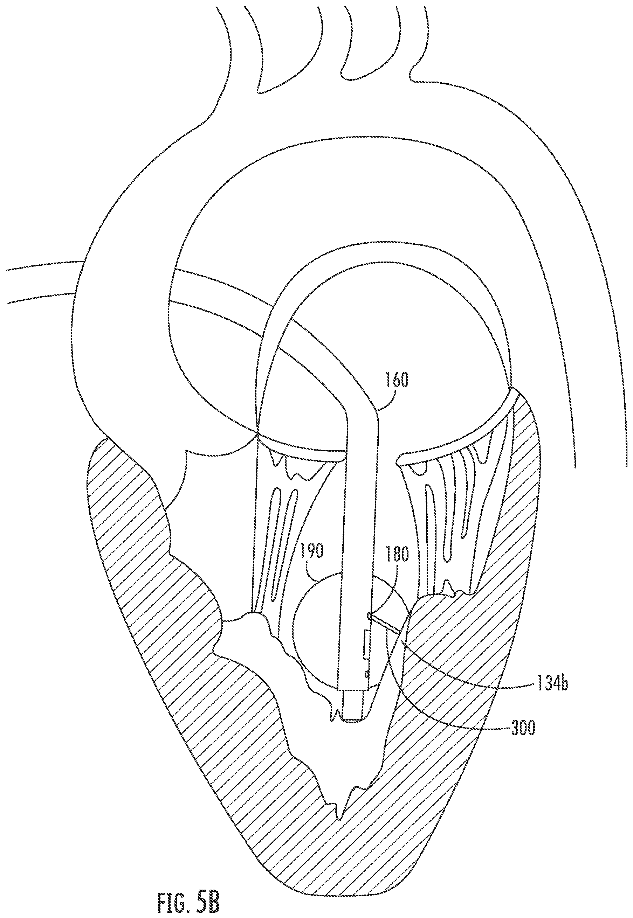

**In** FIG. 5B, the visualization catheter 160 has been rotated so that port 180 is aligned with target tissue of the papillary muscle 134b. The needle 310 may then be advanced through the anchor lumen (such as anchor lumen 410 of FIG. 4A), and into the target tissue. In one embodiment, the balloon 190 may comprise a plurality of longitudinally disposed segments, enabling the needle 310 to extend through the segments into the tissue.

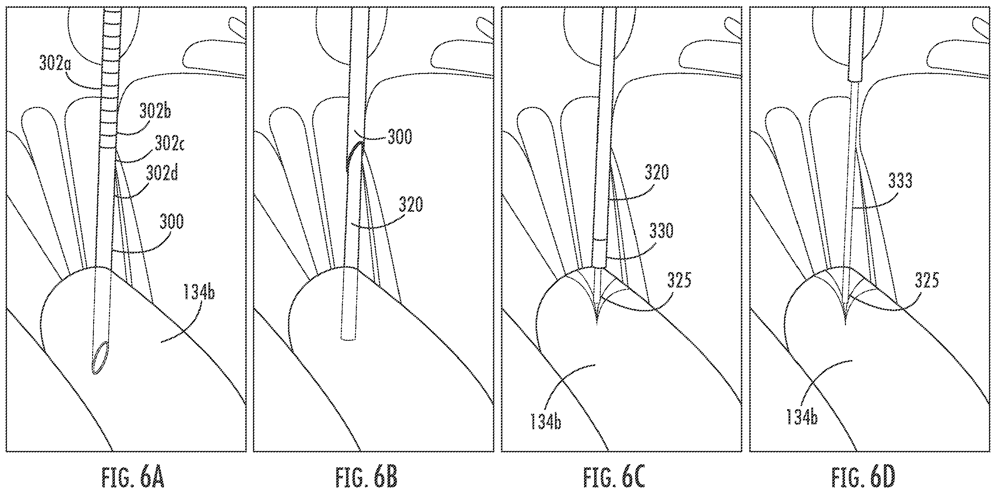

Referring briefly to FIGS. 6A-6D, operation of the anchor deployment system for needle insertion is shown in detail. In FIG. 6A, the needle 310 is advanced into papillary muscle 134b. In one embodiment, the needle 310 may include echogenic depth markings 302a-302d, which may provide visual feedback to a surgeon as to the depth of penetration of the needle 310 in the papillary muscle 134b. In one embodiment, needle 310 includes a sharpened distal tip that cuts an opening into the papillary tissue 134b.

In FIG. 6B, the anchor catheter 320 may be forwarded into the opening made by the needle 310, and the needle 310 may be proximally withdrawn. In FIG. 6C, the anchor 325 is pushed out of anchor catheter 320 by action of a push tube 330 into the papillary muscle 134b. A suture (not visible in FIG. 6C) is coupled to the anchor 325 and extends proximally through the push tube 330, or along the exterior of the push tube 330 through the anchor catheter 320. FIG. 6D illustrates that, when the anchor catheter 320 is proximally withdrawn, the suture 333 and anchor 325 remain coupled to the papillary muscle 134b within the cardiac cavity.

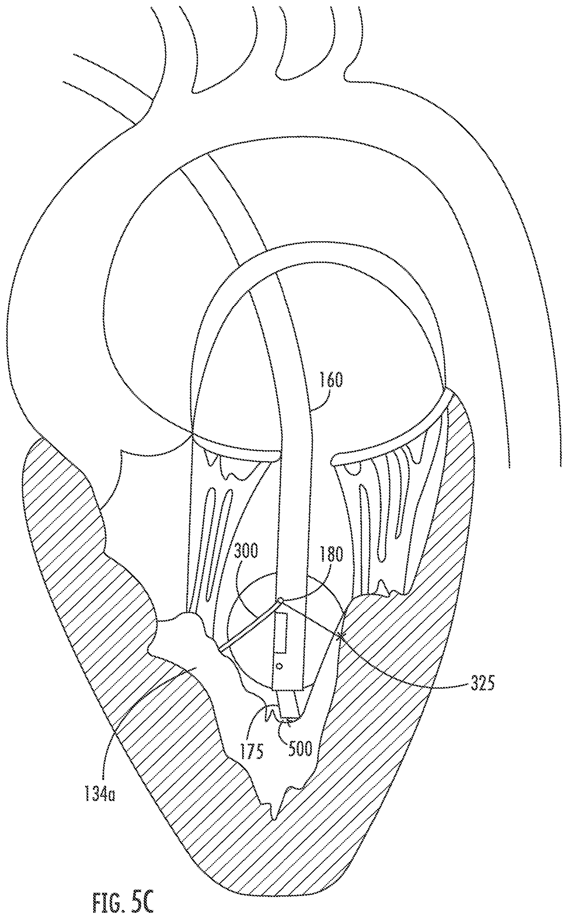

Referring now to FIG. 5C, once the first anchor 325 is positioned, the visualization catheter 160 may be rotated toward papillary muscle 134a, and the needle 310 may again be forwarded through a port 180 towards target tissue of the papillary muscle 134a. Because the depth adjustment mechanism 175 retains the height of the visualization catheter relative to the inner wall of the apex 500 of the heart, the second deployed anchor is placed along the same circumferential axis about the visualization catheter 160, thereby reducing vector forces that may reduce the efficacy of the cardiac repair.

In some embodiments, as described above, the visualization catheter may include multiple ports distributed longitudinally along the distal portion of the visualization catheter. Providing multiple ports may allow deployment of multiple parallel cinching structures that may improve the integrity of cardiac restructuring, although the present disclosure is not so limited.

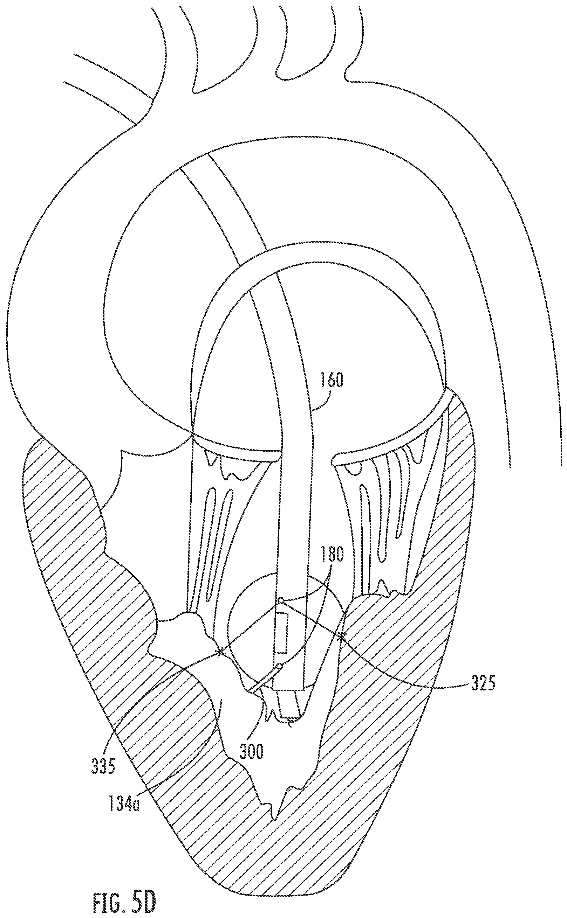

By way of example, In FIG. 5D, anchor 335 is shown driven into cardiac tissue, such as the papillary muscle 134a, opposite of anchor 325. In one embodiment, following deployment of anchor 335a, needle 310 may be advanced, with an additional anchor, through a second one of the ports 180, providing second anchoring of the papillary muscle 134a.

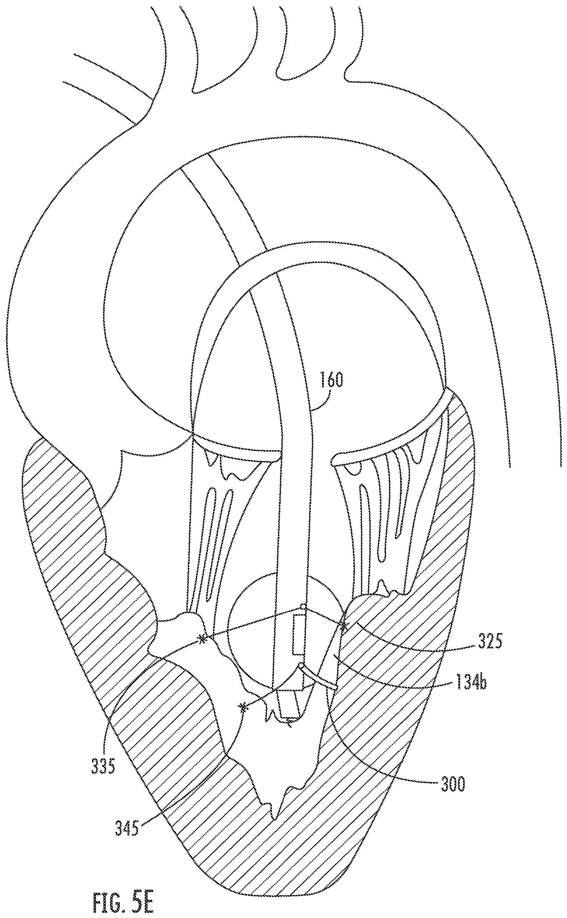

As shown in FIG. 5E, visualization catheter 160 may again be rotated towards papillary muscle 134b, for placement of a fourth anchor opposite anchor 345 using needle 310 (placed in FIG. 5D). Like anchor pair 325, 335, the newly deployed anchor is circumferentially aligned about the visualization catheter 160 with anchor 345.

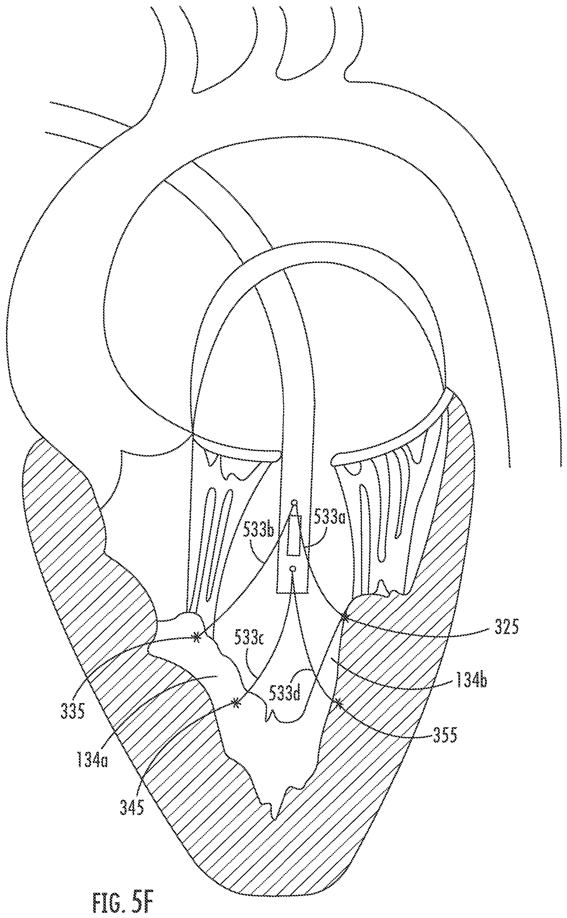

FIG. 5F illustrates four deployed anchors 325, 335, 345, and 355. Each anchor is coupled to one of a plurality of sutures 533a-533d. In one embodiment, following placement of the anchors, the depth adjustment mechanism may be proximally withdrawn into the visualization catheter. The sutures 533a-533d may be withdrawn proximally to pull together papillary muscles 134a, 134b to restore papillary structure to approximate that of a healthy heart. A clamping tool may be advanced through a working channel of the visualization catheter to bind together suture pairs to retain the reconfigured structure. In an alternative approach, the visualization catheter may be removed, then a second catheter containing the clamping tool may be strung down over 2 or more of the sutures.

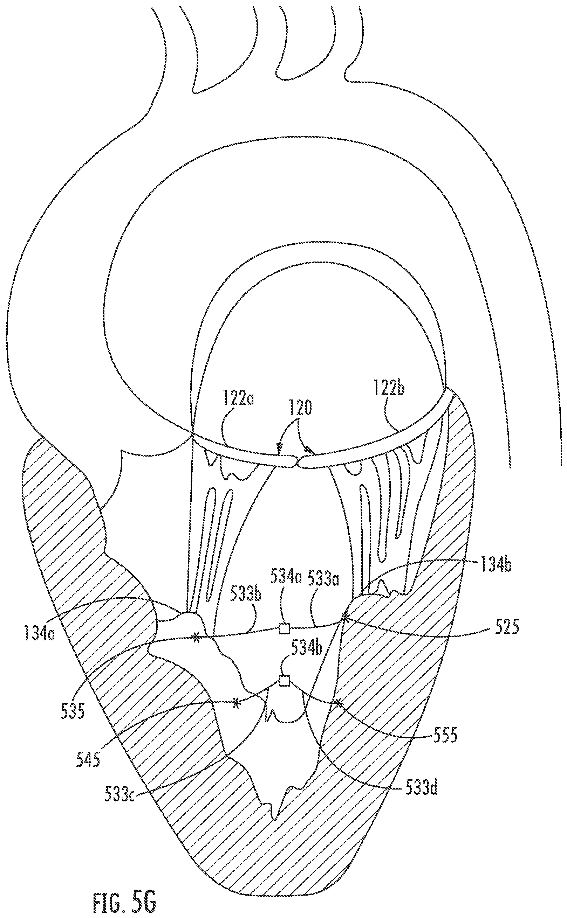

FIG. 5G illustrates a reconfigured papillary structure, wherein the sutures 533a and 533b, coupled to anchors 525, 535 are joined by clamp 534a (which may be, for example, a resistive weld or the like), and sutures 533c and 533d, coupled to anchors 555, 545 are joined by clamp 534b. As shown in FIG. 5G, pulling together the papillary muscles 134a, 134b in this manner draws together the leaflets 122a, 122b of the mitral valve 120 to restore heart function.

Although FIGS. 5A-5G described placement of multiple anchors 525, 535, 545 and 555 in a particular order, and coupled in a particular pattern, the present disclosure is not limited to the disclosed order of placement or pattern of connection between anchors. Rather, it is appreciated that the order, placement, and pattern of connectivity may vary depending upon a variety of factors including but not limited to the anatomy and diseased state of the heart under treatment.

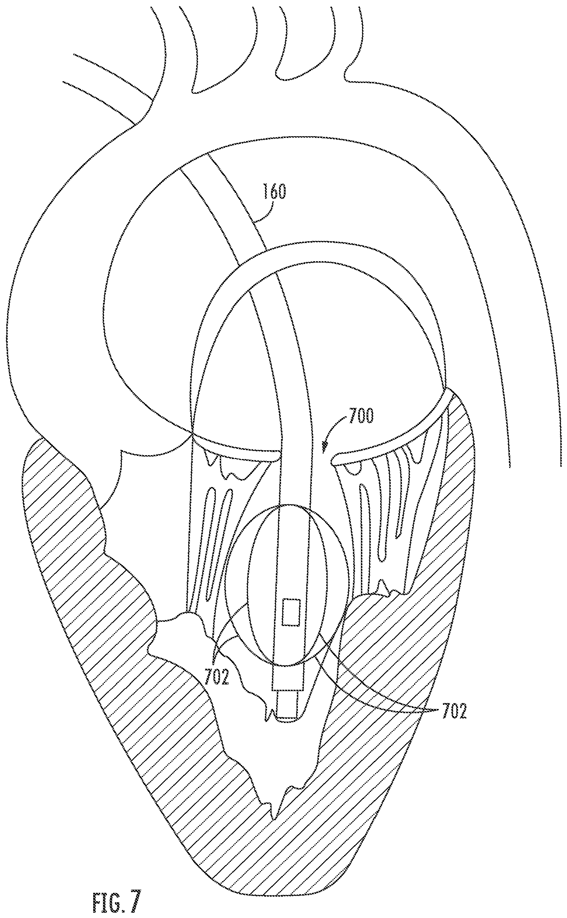

Various embodiments of the disclosed system for delivery of sub-valvular repair components have included a balloon-based retention mechanism, although it is appreciated that various alternative methods for retention mechanisms may be substituted herein by one of skill in the art. For example, FIG. 7 illustrates a spline-based retention system 700, wherein a plurality of splines 702 are longitudinally disposed along a surface of the visualization catheter 160. Each spline may be coupled to the exterior surface of the visualization catheter at a proximal and distal end. The plurality of splines may extend partially around or completely around the visualization catheter. In one embodiment, the splines 702 may be formed of a shape memory material, such as nitinol, that may comprise a linear configuration, wherein the splines lay flush against an exterior surface of the visualization catheter, and an expanded configuration such as that shown in FIG. 7, wherein at least a portion of the splines extend radially from the exterior surface of the visualization catheter 160. In one embodiment, the splines may be formed to be biased in the expanded configuration, and heat activated to expand to the biased configuration.

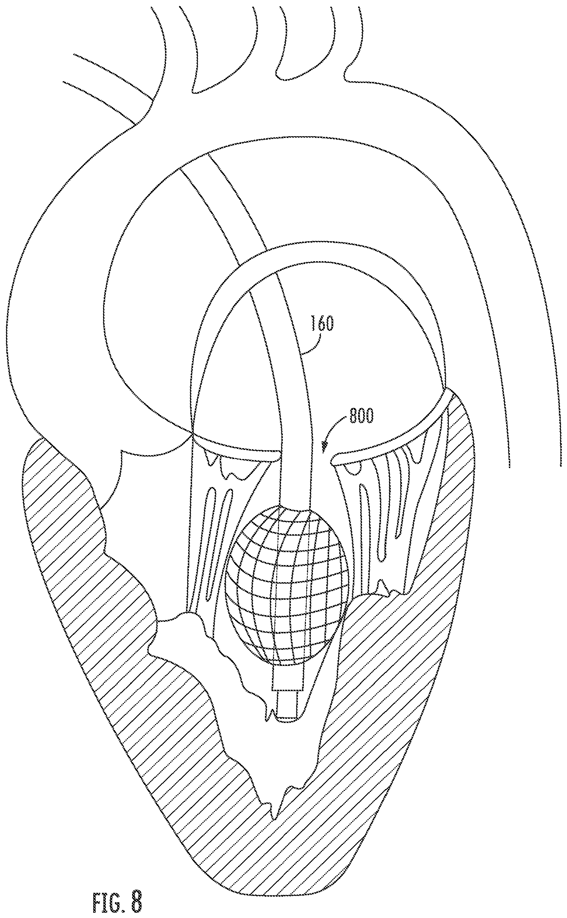

FIG. 8 illustrates another embodiment of a retention mechanism 800, wherein in FIG. 8 the retention mechanism 800 comprises an expandable stent, for example comprising a woven or braided material biased towards an expanded configuration, for example, to secure the visualization catheter 160 during anchor deployment.

Accordingly, a system and method for sub-valvular repair has been shown and described. Various modifications to the implementations described in this disclosure will be readily apparent to those skilled in the art, and the generic principles defined herein can be applied to other implementations without departing from the scope of this disclosure. Thus, the disclosure is not intended to be limited to the implementations shown herein but is to be accorded the widest scope consistent with the appended claims. The word "example" is used exclusively herein to mean "serving as an example, instance, or illustration." Any implementation described herein as an "example" is not necessarily to be construed as preferred or advantageous over other implementations, unless otherwise stated.

Certain features that are described in this specification in the context of separate implementations also can be implemented in combination in a single implementation. Conversely, various features that are described in the context of a single implementation also can be implemented in multiple implementations separately or in any suitable sub-combination. Moreover, although features can be described above as acting in certain combinations and even initially claimed as such, one or more features from a claimed combination can in some cases be excised from the combination, and the claimed combination can be directed to a sub-combination or variation of a sub-combination. Similarly, while operations are depicted in the drawings in a particular order, this should not be understood as requiring that such operations be performed in the particular order shown or in sequential order, or that all illustrated operations be performed, to achieve desirable results. Additionally, other implementations are within the scope of the following claims. In some cases, the actions recited in the claims can be performed in a different order and still achieve desirable results.

It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

## Claims

1. A delivery system (150) including:
a visualization catheter (160) comprising a proximal end (215), a distal end (205), and an elongate tubular body (210) extending from the proximal end (215) to the distal end (205) of the visualization catheter (160), the visualization catheter (160) comprising an imaging device (170) coupled to a distal portion of the visualization catheter (160);
an anchor lumen (410, 420), extending from the proximal end of the visualization catheter (160) through an anchor port (180) disposed on a distal wall of the visualization catheter (160) proximate to its distal end (205); and
**characterised in that** the delivery system (150) further includes an adjustment mechanism (175), translatably disposed within a lumen of the visualization catheter (160), the adjustment mechanism (175) configured to align the anchor port (180) with a tissue target based on feedback provided by the imaging device (170).

2. The delivery system of claim 1, wherein the imaging device (170) comprises an ultrasound transducer.

3. The delivery system of claim 1, wherein the adjustment mechanism (175) includes a distal anchor (176).

4. The delivery system of claim 1, further comprising a retention mechanism (190), disposed about the visualization catheter (160), the retention mechanism (190) comprising a linear configuration wherein the retention mechanism (190) is flush with the visualization catheter (160) and an expanded configuration wherein the retention mechanism (190) extends radially from the visualization catheter (160) towards cardiac tissue.

5. The delivery system of claim 4, wherein the retention mechanism (190) comprises a balloon, and the balloon is coupled to an inflation lumen of the visualization catheter.

6. The delivery system of claim 4, wherein the retention mechanism (190) comprises a plurality of expandable splines.

7. The delivery system of claim 4, wherein the retention mechanism (190) comprises an expandable stent.

8. The delivery system of any of claims 4-7, wherein the retention mechanism (190) is disposed about at least a portion of the imaging device (170).

9. The delivery system of any of claims 1-7, further comprising:
a needle (310), translatably disposed within the anchor lumen, the needle (310) comprising a sharpened distal tip (311) and having a needle lumen (312) extending therethrough;
an anchor delivery catheter (320), translatably disposed within the needle lumen (312), the anchor delivery catheter (320) having a proximal end, a distal end and an anchor lumen extending therethrough;
an anchor (325, 335, 345, 355, 525, 535, 545, 555), disposed within the anchor delivery catheter (320); and
a push rod (330), translatably disposed within the anchor delivery catheter (320) proximally of the anchor (325, 335, 345, 355, 525, 535, 545, 555), the push rod (325) configured to advance the anchor (325, 335, 345, 355, 525, 535, 545, 555) through the distal end of the anchor delivery catheter (320) into the tissue target.

10. The delivery system of claims 9, further comprising:
a plurality of anchors (325, 335, 345, 355, 525, 535, 545, 555), disposed within the anchor delivery catheter (320), wherein each anchor (325, 335, 345, 355, 525, 535, 545, 555) is coupled to a proximal end of one of a plurality of sutures (333, 533), the proximal end of the sutures (333, 533) extending proximally through the anchor lumen;
wherein the push rod (330) is configured to advance the plurality of anchors (325, 335, 345, 355, 525, 535, 545, 555) and the plurality of sutures (333, 533) through the distal end of the anchor delivery catheter (320) into different tissue targets; and
a clamping mechanism (534), translatably disposed within a working channel of the visualization catheter (160), the clamping mechanism (534) configured to join at least two of the plurality of sutures (333, 533).

## Patentansprüche

1. Abgabesystem (150) aufweisend:
einen Visualisierungskatheter (160) umfassend ein proximales Ende (215), ein distales Ende (205) und einen langgestreckten rohrförmigen Körper (210), der von dem proximalen Ende (215) zu dem distalen Ende (205) des Visualisierungskatheters (160) verläuft, wobei der Visualisierungskatheter (160) eine Bildgebungsvorrichtung (170) umfasst, die an einen distalen Abschnitt des Visualisierungskatheters (160) gekoppelt ist;
ein Ankerlumen (410, 420), das von dem proximalen Ende des Visualisierungskatheters (160) durch eine Ankeröffnung (180), die an einer distalen Wand des Visualisierungskatheters (160) nahe seinem distalen Ende (205) angeordnet ist, verläuft; und
**dadurch gekennzeichnet, dass** das Abgabesystem (150) ferner einen Einstellmechanismus (175) enthält, der verschiebbar innerhalb eines Lumens des Visualisierungskatheters (160) angeordnet ist, wobei der Einstellmechanismus (175) dafür gestaltet ist, die Ankeröffnung (180) auf der Grundlage einer von der Bildgebungsvorrichtung (170) bereitgestellten Rückmeldung mit einem Gewebeziel auszurichten.

2. Abgabesystem nach Anspruch 1, wobei die Bildgebungsvorrichtung (170) einen Ultraschallwandler umfasst.

3. Abgabesystem nach Anspruch 1, wobei der Einstellmechanismus (175) einen distalen Anker (176) enthält.

4. Abgabesystem nach Anspruch 1, ferner umfassend einen Rückhaltemechanismus (190), der um den Visualisierungskatheter (160) angeordnet ist, wobei der Rückhaltemechanismus (190) eine lineare Konfiguration, bei der der Rückhaltemechanismus (190) bündig mit dem Visualisierungskatheter (160) ist, und eine erweiterte Konfiguration, bei der der Rückhaltemechanismus (190) radial von dem Visualisierungskatheter (160) in Richtung zu Herzgewebe vorsteht, umfasst.

5. Abgabesystem nach Anspruch 4, wobei der Rückhaltemechanismus (190) einen Ballon umfasst und der Ballon an ein Aufblählumen des Visualisierungskatheters gekoppelt ist.

6. Abgabesystem nach Anspruch 4, wobei der Rückhaltemechanismus (190) eine Vielzahl von expandierbaren Rippen umfasst.

7. Abgabesystem nach Anspruch 4, wobei der Rückhaltemechanismus (190) einen expandierbaren Stent umfasst.

8. Abgabesystem nach einem der Ansprüche 4-7, wobei der Rückhaltemechanismus (190) um wenigstens einen Teil der Bildgebungsvorrichtung (170) angeordnet ist.

9. Abgabesystem nach einem der Ansprüche 1-7, ferner umfassend:
eine Nadel (310), die verschiebbar in dem Ankerlumen angeordnet ist, wobei die Nadel (310) eine scharfe distale Spitze (311) umfasst und ein hindurch verlaufendes Nadellumen (312) aufweist;
einen Ankerabgabekatheter (320), der verschiebbar in dem Nadellumen (312) angeordnet ist, wobei der Ankerabgabekatheter (320) ein proximales Ende, ein distales Ende und ein hindurch verlaufendes Ankerlumenm aufweist;
einen Anker (325, 335, 345, 355, 525, 535, 545, 555), der in dem Ankerabgabekatheter (320) angeordnet ist; und
eine Schubstange (330), die verschiebbar in dem Ankerabgabekatheter (320) proximal zu dem Anker (325, 335, 345, 355, 525, 535, 545, 555) angeordnet ist, wobei die Schubstange (325) dafür gestaltet ist, den Anker (325, 335, 345, 355, 525, 535, 545, 555) durch das distale Ende des Ankerabgabekatheters (320) in das Gewebeziel vorzuschieben.

10. Abgabesystem nach Anspruch 9, ferner umfassend:
eine Vielzahl von Ankern (325, 335, 345, 355, 525, 535, 545, 555), die in dem Ankerabgabekatheter (320) angeordnet sind, wobei jeder Anker (325, 335, 345, 355, 525, 535, 545, 555) an ein proximales Ende von einer aus einer Vielzahl von Nahtfäden (333, 533) gekoppelt ist, wobei die proximalen Enden der Nahtfäden (333, 533) proximal durch das Ankerlumen verlaufen;
wobei die Schubstange (330) dafür gestaltet ist, die Vielzahl von Ankern (325, 335, 345, 355, 525, 535, 545, 555) und die Vielzahl von Nahtfäden (333, 533) durch das distale Ende des Ankerabgabekatheters (320) in verschiedene Gewebeziele vorzuschieben; und
einen Klemmmechanismus (534), der verschiebbar in einem Arbeitskanal des Visualisierungskatheters (160) angeordnet ist, wobei der Klemmmechanismus (534) dafür gestaltet ist, wenigstens zwei der Vielzahl von Nahtfäden (333, 533) zu verbinden.

## Revendications

1. Système d'administration (150) comprenant :
un cathéter de visualisation (160) comprenant une extrémité proximale (215), une extrémité distale (205), et un corps tubulaire allongé (210) s'étendant de l'extrémité proximale (215) à l'extrémité distale (205) du cathéter de visualisation (160), le cathéter de visualisation (160) comprenant un dispositif d'imagerie (170) couplé à une partie distale du cathéter de visualisation (160) ;
une lumière d'ancrage (410, 420), s'étendant de l'extrémité proximale du cathéter de visualisation (160) à travers un orifice d'ancrage (180) disposé sur une paroi distale du cathéter de visualisation (160) à proximité de son extrémité distale (205) ; et
**caractérisé en ce que** le système d'administration (150) comprend en outre un mécanisme de réglage (175), disposé en translation à l'intérieur d'une lumière du cathéter de visualisation (160), le mécanisme de réglage (175) étant configuré pour aligner l'orifice d'ancrage (180) avec une cible tissulaire sur la base d'un retour d'information fourni par le dispositif d'imagerie (170).

2. Système d'administration selon la revendication 1, le dispositif d'imagerie (170) comprenant un transducteur à ultrasons.

3. Système d'administration selon la revendication 1, le mécanisme de réglage (175) comprenant un ancrage distal (176).

4. Système d'administration selon la revendication 1, comprenant en outre un mécanisme de retenue (190), disposé autour du cathéter de visualisation (160), le mécanisme de retenue (190) comprenant une configuration linéaire, le mécanisme de retenue (190) affleurant le cathéter de visualisation (160) et une configuration étendue, le mécanisme de retenue (190) s'étendant radialement à partir du cathéter de visualisation (160) vers le tissu cardiaque.

5. Système d'administration selon la revendication 4, le mécanisme de retenue (190) comprenant un ballonnet, et le ballonnet étant couplé à une lumière de gonflage du cathéter de visualisation.

6. Système d'administration selon la revendication 4, le mécanisme de retenue (190) comprenant une pluralité de cannelures extensibles.

7. Système d'administration selon la revendication 4, le mécanisme de retenue (190) comprenant une endoprothèse extensible.

8. Système d'administration selon l'une quelconque des revendications 4 à 7, le mécanisme de retenue (190) étant disposé autour d'au moins une partie du dispositif d'imagerie (170).

9. Système d'administration selon l'une quelconque des revendications 1 à 7, comprenant en outre :
une aiguille (310), disposée en translation dans la lumière d'ancrage, l'aiguille (310) comprenant une pointe distale aiguisée (311) et une lumière d'aiguille (312) s'étendant à travers elle ;
un cathéter de pose d'ancrage (320), disposé en translation dans la lumière d'aiguille (312), le cathéter de pose d'ancrage (320) ayant une extrémité proximale, une extrémité distale et une lumière d'ancrage s'étendant à travers elle ;
un ancrage (325, 335, 345, 355, 525, 535, 545, 555), disposée à l'intérieur du cathéter de pose d'ancrage (320) ; et
une tige de poussée (330), disposée en translation à l'intérieur du cathéter de pose d'ancrage (320) à proximité de l'ancrage (325, 335, 345, 355, 525, 535, 545, 555), la tige de poussée (325) étant configurée pour faire avancer l'ancrage (325, 335, 345, 355, 525, 535, 545, 555) à travers l'extrémité distale du cathéter de pose d'ancrage (320) jusqu'à la cible tissulaire.

10. Système d'administration selon la revendication 9, comprenant en outre :
une pluralité d'ancrages (325, 335, 345, 355, 525, 535, 545, 555), disposés à l'intérieur du cathéter de pose d'ancrage (320), chaque ancrage (325, 335, 345, 355, 525, 535, 545, 555) étant couplé à une extrémité proximale de l'une d'une pluralité de sutures (333, 533), l'extrémité proximale des sutures (333, 533) s'étendant proximalement à travers la lumière d'ancrage ;
la tige de poussée (330) étant configurée pour faire avancer la pluralité d'ancrages (325, 335, 345, 355, 525, 535, 545, 555) et la pluralité de sutures (333, 533) à travers l'extrémité distale du cathéter de pose d'ancrages (320) dans différentes cibles tissulaires ; et
un mécanisme de serrage (534), disposé en translation à l'intérieur d'un canal de travail du cathéter de visualisation (160), le mécanisme de serrage (534) étant configuré pour joindre au moins deux de la pluralité de sutures (333, 533).
